# EUROPÄISCHE PATENTSCHRIFT

(11) **EP 1 127 549 B1**
(45) Veröffentlichungstag und Bekanntmachung des Hinweises auf die Patenterteilung: **09.06.2004**
(21) Anmeldenummer: 01101272.1
(22) Anmeldetag: 19.01.2001
(51) Int. Cl.: A61B 6/14

(54) **Röntgeneinrichtung und medizinischer Arbeitsplatz für die Diagnostik und für chirurgische Eingriffe im Kopf- und Kieferbereich eines Patienten**
X-ray appliance and medical operating post for the diagnosis and surgical interventions in the head and in the jaw of a patient
Appareil de radiographie et poste opérateur pour la diagnose et pour interventions chirurgicales sur la tête et sur la mâchoire d'un patient

(30) Priorität: 22.02.2000 DE 10008053
(43) Veröffentlichungstag der Anmeldung: 29.08.2001
(73) Patentinhaber: SIEMENS AKTIENGESELLSCHAFT, 80333 München (DE)
(72) Erfinder: Graumann, Rainer, Dr., 91315 Höchstadt (DE)

(56) Entgegenhaltungen:
- EP-A- 0 641 545
- EP-A- 0 857 461
- EP-A- 0 910 990
- WO-A-97/40766
- DE-A- 19 754 670
- US-A- 5 253 171
- US-A- 5 666 392

## Beschreibung

Die Erfindung betrifft eine Röntgeneinrichtung für radiologische Aufnahmen im Kopf- und Kieferbereich eines Patienten mit einer Röntgenstrahlenquelle und einem Röntgenstrahlendetektor zur Aufnahme von 2D-Projektionen vom Kopf- oder Kieferbereich des Patienten. Die Erfindung betrifft außerdem einen medizinischen Arbeitsplatz für die Diagnostik und für chirurgische Eingriffe im Mund-, Kiefer- oder Gesichtsbereich eines Patienten, aufweisend eine derartige Röntgeneinrichtung.

Für die Diagnose und Planung von kieferchirurgischen Eingriffen, Zahnimplantierungen oder Eingriffen im Gesichtsbereich eines Patienten werden je nach Bedarf und medizinischer Notwendigkeit Röntgenaufnahmen des Kiefers, Röntgenprojektionsaufnahmen von Einzelzähnen, Röntgenpanoramaaufnahmen des Kiefers oder Computertomographieaufnahmen zur Gewinnung von 3D-Bildern von Gewebebereichen des Kopfes oder des Kiefers durchgeführt. Letztere werden aus Kostengründen nur in begründeten Einzelfällen z. B. für die Planung von Zahnimplantationen oder bei rekonstruktiven Eingriffen im Gesichts-/Schädelbereich erstellt, bei denen eine Eingriffsplanung anhand von 3D-Bildern unerlässlich ist. Da es sich bei einem Röntgen-Computertomographen um ein relativ teures Bildaufnahmegerät handelt, verfügen nur die wenigsten Kieferchirurgen über ein derartiges Gerät, weshalb die Planung und Durchführung eines kieferchirurgischen Eingriffes in der Regel eine Vielzahl von nicht unmittelbar aufeinanderfolgenden und vom Kieferchirurgen durchführbaren Prozessschritten erforderlich macht. Für die Planung und Durchführung einer Zahnimplantation ist es beispielsweise erforderlich,
a) beim Kieferchirurgen Übersichtsaufnahmen vom Kiefer anzufertigen,
b) bei Bedarf Aufnahmen mit einem Röntgen-Computertomographen vom Kiefer bei einem Radiologen durchführen zu lassen,
c) die Computertomographiedaten zum Kieferchirurgen zu transferieren,
d) den Eingriff beim Kieferchirurgen zu planen und
e) den Eingriff am Patienten durchzuführen.

In der DE 40 12 627 A1 ist beispielweise ein Röntgengerät zur Aufnahme räumlicher Röntgenbilder eines Kiefers auf einen Röntgenfilm beschrieben.

Aus der DE 196 36 354 A1 ist eine Vorrichtung zur Durchführung optischer Aufnahmen insbesondere eines Zahnes bekannt.

Mit dem in der DE 197 54 670 A1 beschriebenen Röntgengerät, das eine Trageeinrichtung zum Halten der Röntgenquelle und der Röntgenbildaufnahmeeinrichtung umfasst, können sowohl Teil-CT-Bilder als auch Panoramabilder aufgenommen werden. Entsprechend des eingestellten Modus werden die Röntgenquelle und die Röntgenbildaufnahmeeinrichtung auf verschiedenen Bahnen verstellt.

In der EP 0 910 990 A1 ist ein C-Bogen Röntgengerät beschrieben, mit dem aus einer Serie von 2D-Projektionen vom Körperbereich eines Patienten ein 3D-Bild hergestellt werden kann.

Der Erfindung liegt die Aufgabe zugrunde, eine Röntgeneinrichtung bzw. einen mit einer derartigen Röntgeneinrichtung versehenen medizinischen Arbeitsplatz zur Verfügung zu stellen, welche kostengünstig ausführbar ist und mit welcher auf kostengünstige Weise 3D-Bilder vom Kopf- und Kieferbereich eines Patienten gewonnen werden können.

Nach der Erfindung wird diese Aufgabe gelöst durch eine Röntgeneinrichtung für radiologische Aufnahmen im Kopf- und Kieferbereich eines Patienten mit einer Röntgenstrahlenquelle und einem Röntgenstrahlendetektor, welche derart an einer U-förmig oder C-bogenförmig ausgebildeten Tragevorrichtung einander gegenüberliegend angeordnet sind, dass der Zentralstrahl eines von der Röntgenstrahlenquelle ausgehenden kegelförmigen Röntgenstrahlenbündels annähernd mittig auf den Röntgenstrahlendetektor trifft, dadurch gekennzeichnet, dass
- die Röntgeneinrichtung Mittel zur motorischen Verstellung der Tragevorrichtung um eine Achse zur Aufnahme einer Serie von 2D-Projektionen vom Kopf- oder Kieferbereich des Patienten aufweist, wobei die Achse durch die Tragevorrichtung verläuft und die Tragevorrichtung um die Achse motorisch schwenkbar ist, und
- die Röntgeneinrichtung Mittel zur Erzeugung eines 3D-Bilddatensatzes aus den aufgenommenen 2D-Projektionen umfasst.

Die Aufgabe der Erfindung wird auch gelöst durch eine Röntgeneinrichtung für radiologische Aufnahmen im Kopf- und Kieferbereich eines Patienten mit einer Röntgenstrahlenquelle und einem Röntgenstrahlendetektor, welche derart einander gegenüberliegend angeordnet werden können oder angeordnet sind, dass der Zentralstrahl eines von der Röntgenstrahlenquelle ausgehenden kegelförmigen Röntgenstrahlenbündels annähernd mittig auf den Röntgenstrahlendetektor trifft und mit Mitteln zur Halterung der Röntgenstrahlenquelle und des Röntgenstrahlendetektors, dadurch gekennzeichnet, dass
- die Röntgeneinrichtung Mittel zur motorischen Verstellung der Mittel zur Halterung um eine Achse zur Aufnahme einer Serie von 2D-Projektionen vom Kopf- oder Kieferbereich des Patienten aufweist,
- die Mittel zur Halterung der Röntgenstrahlenquelle und des Röntgenstrahlendetektors zwei Stative umfassen, wobei die Röntgenstrahlenquelle und der Röntgenstrahlendetektor jeweils an einem der Stative angeordnet sind, welche um die Achse und relativ zueinander motorisch verstellbar sind und
- die Röntgeneinrichtung Mittel zur Erzeugung eines 3D-Bilddatensatzes aus den aufgenommenen 2D-Projektionen aufweist.

Die erfindungsgemäße Röntgeneinrichtung weist ähnlich wie Rotationsangiographiegeräte oder verfahrbare C-Bogen-Röntgengeräte eine ein kegelförmiges Röntgenstrahlenbündel aussendende Röntgenstrahlenquelle auf, welche zusammen mit einem Röntgenstrahlendetektor um eine Achse der Röntgeneinrichtung, entlang welcher der Kopf- des zu untersuchenden Patienten in der Regel gelagert ist, verstellt werden kann. Während der Verstellbewegung um die Achse wird eine Serie von 2D-Projektionen aus unterschiedlichen Projektionswinkeln vom Kopf- oder Kieferbereich des Patienten aufgenommen, aus denen ein 3D-Bilddatensatz vom Kopf- oder Kieferbereich des Patienten gewonnen werden kann. Der Aufbau der Röntgeneinrichtung, z. B. der Abstand der Röntgenstrahlenquelle von dem Röntgenstrahlendetektor und der Verstellweg der Röntgenstrahlenquelle und des Röntgenstrahlendetektors, ist dabei den Erfordernissen im Mund-, Kiefer- und Gesichtsbereich von Patienten angepasst. Die Röntgeneinrichtung kann somit verhältnismäßig klein gehalten werden. Vorzugsweise weisen die Röntgenstrahlenquelle und der Röntgenstrahlendetektor maximal einen Abstand von einem Meter voneinander auf. Da bei der erfindungsgemäßen Röntgeneinrichtung relativ preisgünstig erhältliche und bewährte Komponenten verwendet werden können, kann die Röntgeneinrichtung insgesamt kostengünstig ausgeführt werden. Somit sind die wirtschaftlichen Bedingungen für die Ausstattung einer kieferchirurgischen Praxis mit einem derartigen Röntgengerät günstig, so dass ein Kieferchirurg die Bildaufnahme, die Eingriffsplanung und den Eingriff am Patienten in direkt aufeinanderfolgenden Schritten ohne Zeitverzögerung durch mehrmalige Sitzungen durchführen kann.

Die Mittel zur Halterung der Röntgenstrahlenquelle und des Röntgenstrahlendetektors umfassen entweder zwei Stative, wobei die Röntgenstrahlenquelle und der Röntgenstrahlendetektor jeweils an einem der Stative angeordnet sind, welche um die Achse der Röntgeneinrichtung und relativ zueinander motorisch verstellbar sind. Derartige Stative können beispielsweise an einer deckenmontierten, kreisförmig um die Achse verlegten Schiene geführt sein.

Alternativ ist vorgesehen, dass die Röntgenstrahlenquelle und der Röntgenstrahlendetektor an einer U-förmig oder C-bogenförmig ausgebildeten Tragevorrichtung angeordnet sind, wobei die Tragevorrichtung um die durch die Tragevorrichtung verlaufende Achse motorische schwenkbar ist. Die Tragevorrichtung kann dabei deckenmontiert um eine vertikal verlaufende Achse oder an einer Halterung angeordnet um eine horizontal verlaufende Achse schwenkbar sein.

Eine besonders bevorzugte Ausführungsform der Erfindung sieht vor, dass die motorische Verstellung der Stative bzw. die motorische Schwenkung der Tragevorrichtung durch wenigstens einen digital gesteuerten Antrieb bewirkt wird. Der vorzugsweise softwaregesteuerte Antrieb, welcher nach einer Ausführungsform der Erfindung einen Schrittmotor umfasst, ermöglicht eine präzise Verstellung der Stative relativ zueinander bzw. eine präzise Schwenkung der Tragevorrichtung, wobei verschiedene Stellungen der Stative bzw. der Tragevorrichtung mit hoher Genauigkeit wiederholt angefahren werden können. Da die Röntgeneinrichtung insgesamt relativ klein gehalten werden kann, weist der Aufbau der Röntgeneinrichtung eine hohe Steifigkeit auf, so dass die Verstellbewegungen des an den Stativen bzw. des an der Tragevorrichtung angeordneten, die Röntgenstrahlenquelle und den Röntgenstrahlendetektor umfassenden Röntgensystems reproduzierbar sind.

Eine Variante der Erfindung sieht vor, dass der Röntgenstrahlendetektor relativ zu seinem Stativ bzw. relativ zu der Tragevorrichtung in Richtung des Zentralstrahls verstellbar ist. Auf diese Weise kann der Röntgenstrahlendetektor relativ nahe am Kopf des Patienten platziert und somit die Größe des Bildfeldes für den jeweiligen Untersuchungsfall optimal eingestellt werden. Die Röntgenstrahlenquelle und der Röntgenstrahlendetektor bewegen sich daher bei der Aufnahme einer Serie von 2D-Projektionen in der Regel asymmetrisch um die Achse bzw. den Kopf des Patienten.

Die Aufgabe der Erfindung wird auch gelöst durch einen medizinischen Arbeitsplatz für die Diagnostik und für chirurgische Eingriffe im Mund-, Kiefer- oder Gesichtsbereich eines Patienten aufweisend eine Röntgeneinrichtung, wie vorstehend erwähnt, eine Patientenlagerungseinrichtung und Mittel zur Bestimmung der Positionen der Röntgeneinrichtung und der Patientenlagerungseinrichtung relativ zueinander, wobei die Röntgeneinrichtung und die Patientenlagerungseinrichtung in definierter Weise relativ zueinander angeordnet und in definierter Weise relativ zueinander mechanisch verstellbar sind.

Die Aufgabe wird auch gelöst durch einen medizinischen Arbeitsplatz für die Diagnostik und für chirurgische Eingriffe im Mund-, Kiefer- oder Gesichtsbereich eines Patienten, aufweisend eine Röntgeneinrichtung, wie vorstehend erwähnt, eine Patientenlagerungseinrichtung und ein Navigationssystem zur Bestimmung der Positionen der Röntgeneinrichtung und der Patientenlagerungsvorrichtung relativ zueinander.

Beide Ausführungsformen des medizinischen Arbeitsplatzes erlauben es einem Kieferchirurgen durch die Kenntnis der räumlichen Beziehung zwischen der Röntgeneinrichtung und somit dem mit der Röntgeneinrichtung erzeugten 3D-Bilddatensatz und der Patientenlagerungseinrichtung, auf welcher der Patient gelagert und in der Regel fixiert ist, die anhand der 3D-Bilddaten erzielten Planungsergebnisse in einfacher und bequemer Weise unmittelbar auf den Patienten zu übertragen und den Eingriff durchzuführen. Durch die bekannte räumliche Beziehung zwischen dem 3D-Bilddatensatz und der Patientenlagerungsvorrichtung kann bei Verstellungen der Patientenlagerungseinrichtung relativ zu der Röntgeneinrichtung und somit relativ zu dem 3D-Bilddatensatz im Zuge des Eingriffs die Darstellung erzeugter 3D-Bilder der veränderten Lage des Patienten automatisch angepasst werden.

Eine Variante der Erfindung sieht vor, mit einem Navigationssystem nicht nur die Position der Röntgeneinrichtung und/oder der Patientenlagerungsvorrichtung, sondern auch die Position wenigstens eines bei dem kieferchirurgischen Eingriff verwendeten medizinischen Instrumentes zu bestimmen, wodurch die Abbildung des Instrumentes in ein mit der Röntgeneinrichtung gewonnenes Bild zur Unterstützung des chirurgischen Eingriffes ermöglicht wird. Auf diese Weise kann der Chirurg ein in den Körper des Patienten eingedrungenes Instrument anhand der auf einem Sichtgerät dargestellten Bildinformationen navigieren, wodurch sich eine sinnvolle Unterstützung des chirurgischen Eingriffes ergibt.

Ausführungsbeispiele der Erfindung sind in den beigefügten schematischen Zeichnungen dargestellt. Es zeigen:
- Fig. 1: einen kieferchirurgischen Arbeitsplatz mit einem Röntgensystem, welches zwei in einer deckengehängten Schiene angeordnete Stative umfasst, und mit einem Navigationssystem,
- Fig. 2: den Verlauf der deckengehängten Schiene aus Fig. 1,
- Fig. 3: einen kieferchirurgischen Arbeitsplatz mit einem an einem deckengehängten C-Bogen angeordneten Röntgensystem und mit einem Navigationssystem, und
- Fig. 4: einen kieferchirurgischen Arbeitsplatz mit einem an einer U-förmigen Tragevorrichtung angeordneten Röntgensystem.

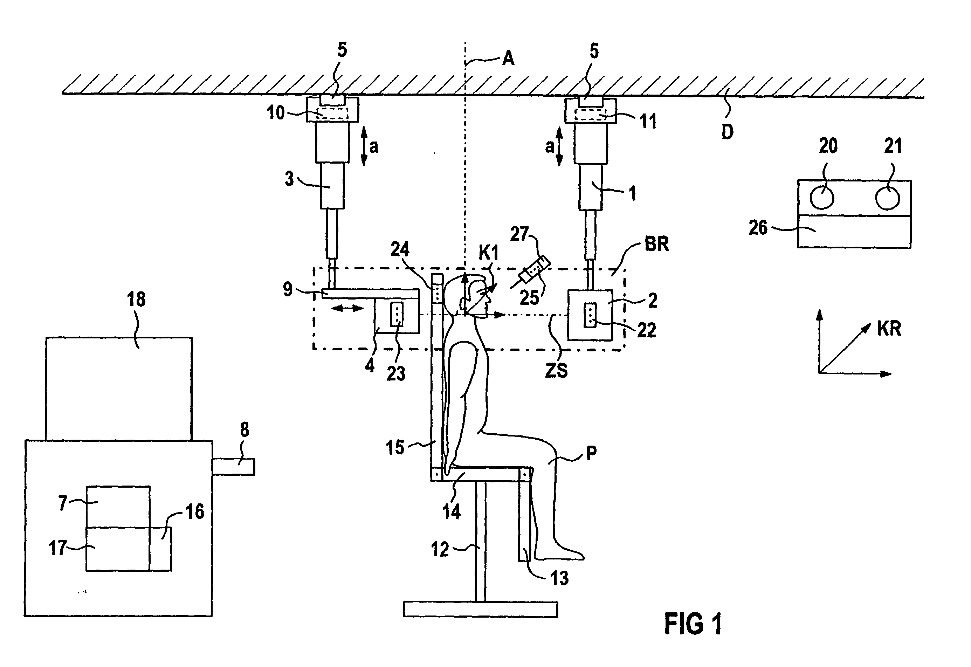

In Fig. 1 ist ein erfindungsgemäßer medizinischer Arbeitsplatz für die Diagnostik und für chirurgische Eingriffe im Mund-, Kiefer- oder Gesichtsbereich eines Patienten P gezeigt. Der Arbeitsplatz umfasst eine Röntgeneinrichtung für radiologische Aufnahmen vom Kopf- und Kieferbereich des Patienten P, eine Patientenlagerungseinrichtung und ein Navigationssystem.

Die Röntgeneinrichtung weist eine an einem Stativ 1 angeordnete Röntgenstrahlenquelle 2 und einen an einem Stativ 3 angeordneten Röntgenstrahlendetektor 4 auf, bei dem es sich beispielsweise um einen Röntgenbildverstärker oder einen aSi-Flachbilddetektor handeln kann. Die Röntgenstrahlenquelle 2 und der Röntgenstrahlendetektor 4 sind derart an den Stativen 1 bzw. 3 angeordnet, dass ein von der Röntgenstrahlenquelle 2 ausgehender Zentralstrahl ZS eines kegelförmigen Röntgenstrahlenbündels annäherungsweise mittig auf den Eingangsschirm des Röntgenstrahlendetektors 4 trifft.

Die Stative 1 und 3 sind im wesentlichen gleichartig ausgeführt und in einer deckengehängten Schiene 5 angeordnet. Bei den Stativen 1 und 3 handelt es sich im Falle des vorliegenden Ausführungsbeispiels um Teleskopstative, welche mittels nicht näher dargestellter elektrischer Antriebe vertikal in die Richtungen der Doppelpfeile a verstellbar sind. Auf diese Weise können die Röntgenstrahlenquelle 2 und der Röntgenstrahlendetektor 4 für radiologische Aufnahmen in einen in Fig. 1 mit gestrichelten Linien angedeuteten Behandlungsraum BR bzw. aus dem Behandlungsraum BR gebracht werden. Die Verstellung der Stative 1, 3 wird von einem Steuerrechner 7 der Röntgeneinrichtung gesteuert und erfolgt vorzugsweise synchron, so dass die Röntgenstrahlenquelle 2 und der Röntgenstrahlendetektor 4 stets relativ zueinander ausgerichtet bleiben. Die vertikale Verstellung der Stative 1, 3 kann von einem Bedienpult 8 durch eine Bedienperson vorgenommen werden. Im Unterschied zu der Anordnung der Röntgenstrahlenquelle 2 an dem Stativ 1 ist der Röntgenstrahlendetektor 4 derart an einer an dem Stativ 3 angebrachten Verstelleinrichtung 9 angeordnet, dass er in Richtung des Zentralstrahles ZS des Röntgenstrahlenbündels in geometrisch bestimmter Weise verschieblich ist. Auf diese Weise kann die Größe des Bildfeldes der jeweiligen Aufnahmesituation bei der Gewinnung von 2D-Projektionen angepasst werden.

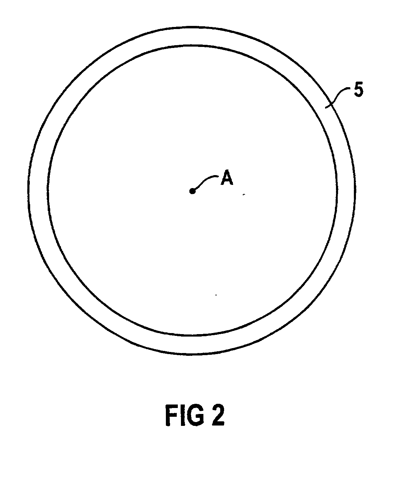

Die Stative 1 und 3 können mittels digital softwaregesteuerter elektrischer Antriebe in Form zweier Schrittmotoren 10, 11 in der Schiene 5 verstellt werden, wobei sich die Stative 1, 3 um eine vertikal verlaufende Achse A des Röntgensystems kreisförmig bewegen. Fig. 2 zeigt stark schematisiert die kreisförmig um die Achse A geführte, an der Decke D montierte Schiene 5. Die Verstellung der Stative 1, 3 in der Schiene 5 wird wiederum von dem Steuerrechner 7 derart gesteuert, dass die Verstellung der Röntgenstrahlenquelle 2 und des Röntgenstrahlendetektors 4 synchron erfolgt und die Röntgenstrahlenquelle 2 und der Röntgenstrahlendetektor 4 relativ zueinander ausgerichtet bleiben.

Der Patient P ist auf einer Patientenlagerungsvorrichtung in Form eines Patientenstuhles 12 gelagert. Der Patientenstuhl 12 ist im Falle des vorliegenden Ausführungsbeispiels derart ausgeführt, dass seine Elemente 13, 14 und 15 in an sich bekannter Weise relativ zueinander motorisch verstellt werden können. Der Patient P kann demnach in verschiedene Stellungen relativ zu der Röntgeneinrichtung gebracht werden. Vorzugsweise ist der Patient P auf dem Patientenstuhl 12 fixiert, so dass er seine Lage auf dem Patientenstuhl 12 während der Behandlung nicht verändern kann.

Zur Aufnahme einer Serie von 2D-Projektionen von Kopf- bzw. Kieferbereich des Patienten P werden die Stative 1 und 3 gesteuert durch den Steuerrechner 7 mit den Schrittmotoren 10, 11 synchron um die Achse A verstellt. In der Regel wird dabei der Röntgenstrahlendetektor 4 asymmetrisch zu der Röntgenstrahlenquelle 2 verstellt. Die bei der Verstellung des Röntgensystems unter verschiedenen Projektionswinkeln aufgenommenen 2D-Projektionen werden in einem Bildspeicher 16 der Röntgeneinrichtung zwischengespeichert und einem Bildrechner 17 zur Erzeugung eines 3D-Bilddatensatzes zur Verfügung gestellt. Die für die Erzeugung eines 3D-Bilddatensatzes erforderlichen Projektionsgeometrien, worunter die Positionen der Röntgenstrahlenquelle 2 und des Röntgenstrahlendetektors 4 sowie die Projektionswinkel bei den verschiedenen 2D-Projektionen in dem willkürlich wählbaren Röntgenkoordinatensystem K1 verstanden werden, erhält der Bildrechner 17 direkt von dem Steuerrechner 7. Die Positionsdaten der Röntgenstrahlenquelle 2 und des Röntgenstrahlendetektors 4, welche in geometrisch bestimmter Weise an den Stativen 1, 3 und um die Achse A angeordnet sind, berechnet der Steuerrechners 7 aus den Steuerdaten der Schrittmotoren 10, 11. Die Ausgangsstellung der Röntgenstrahlenquelle 2 und des Röntgenstrahlendetektors 4 ist dem Steuerrechner 7 aus den Steuerdaten der die vertikale Verstellung der Teleskopstative 1, 3 bewirkenden elektrischen Antrieb, aus den Steuerdaten der Schrittmotoren 10, 11 sowie aus den Einstelldaten der Verstelleinrichtung 9 bekannt. Aus dem in an sich bekannter Weise erzeugten 3D-Bilddatensatz kann der Bildrechner 17 verschiedene 3D-Bilder vom Kopf- bzw. Kieferbereich des Patienten P erzeugen und auf einer Anzeigeeinrichtung 18 darstellen.

Anhand der 3D-Bilder vom Kopf- bzw. Kieferbereich des Patienten P kann ein in Fig. 1 nicht dargestellter Kieferchirurg sofort im Anschluss an die Bildaufnahme den ggf. an dem Patienten P vorzunehmenden Eingriff planen. Dabei ist es vorteilhaft, dass die Lage des erzeugten 3D-Bilddatensatzes und der daraus erzeugter 3D-Bilder in dem Röntgenkoordinatensystem K1 anhand der bekannten Positionen der Röntgenstrahlenquelle 2 und des Röntgenstrahlendetektors 4 bei der Aufnahme der 2D-Projektionen ebenfalls bekannt sind. Auf diese Weise kann der Kieferchirurg die Ergebnisse der Planung direkt auf den Patienten P übertragen und den Eingriff sofort an dem Patienten P vornehmen. Eine derartige Planung besteht beispielsweise in der Festlegung des Ortes und der Orientierung einer Bohrung, welche zur Aufnahme eines Stiftes für ein Zahnimplantat vorgesehen ist. Anhand eines 3D-Bildes kann der Arzt die für die Bohrung erforderlichen Daten festlegen und im Anschluss die Bohrung entsprechend am Kiefer des Patienten P vornehmen.

Im Falle des vorliegenden Ausführungsbeispiels wird außerdem mit Hilfe eines Navigationssystems die räumliche Beziehung zwischen der Röntgeneinrichtung, dem Patientenstuhl 12, auf dem der Patienten P fixiert ist, und einem kieferchirurgischen Instrument 27 erfasst, um bei Verstellungen des Patientenstuhls 12 relativ zu der Röntgeneinrichtung die Bildgebung entsprechend anpassen zu können und das Instrument 27 in ein 3D-Bild einblenden und relativ zu dem Patienten P navigieren zu können.

Das Navigationssystem zur Ermittlung der räumlichen Beziehung zwischen der Röntgeneinrichtung, dem Instrument 27 und dem Patientenstuhl 12 und somit dem auf dem Patientenstuhl 12 fixierten Patienten P ist im Falle des vorliegenden Ausführungsbeispiels ein optisches Navigationssystem, welches Kameras 20, 21 und optische Referenzelemente 22 bis 25 umfasst, welche an den hinsichtlich ihrer Position zu erfassenden Objekten in definierter Weise angeordnet sind und von den Kameras 14, 15 aufgenommen werden. Ein Rechner 26 des Navigationssystems wertet die mit den Kameras 20, 21 aufgenommenen Bilder aus und kann anhand der aufgenommenen Referenzelemente 22 bis 25 die Positionen, d. h. die Lagen und Orientierungen der Referenzelemente 22 bis 25 und somit der entsprechenden Objekte in einem willkürlich wählbaren Referenzkoordinatensystem KR ermitteln.

Im Falle des vorliegenden Ausführungsbeispiels ist das Referenzelement 22 an der Röntgenstrahlenquelle 2, das Referenzelement 23 an dem Röntgenstrahlendetektor 4, das Referenzelement 24 an dem Element 15 des Patientenstuhls 12 und das Referenzelement 25 an dem von dem Kieferchirurgen zu führenden Instrument 27 angeordnet. Der Rechner 26 kann demnach anhand der gewonnenen Kamerabilder jeweils die aktuellen Positionen der Röntgenstrahlenquelle 2, des Röntgenstrahlendetektors 4, des Elementes 15 sowie des Instrumentes 27 in dem Referenzkoordinatensystem KR ermitteln. Der Rechner 26, welcher in nicht dargestellter Weise mit dem Bildrechner 17 verbunden ist, stellt dem Bildrechner 17 jeweils die Daten über die aktuellen Positionen der Röntgenstrahlenquelle 2, des Röntgenstrahlendetektors 4, des Elementes 15, des Patientenstuhls 12 sowie des Instrumentes 27 zur Verfügung. Auf diese Weise kann der Bildrechner 17 eine Beziehung zwischen dem Röntgenkoordinatensystem K1 und dem Referenzkoordinatensystem KR herstellen und auch die Koordinaten des 3D-Bilddatensatzes bezüglich des Referenzkoordinatensystems KR bestimmen. Demnach besteht die Möglichkeit bei einer Veränderung der Stellung des Patientenstuhls 12 und somit des Kopfes des Patienten P den 3D-Bilddatensatz mit der neuen Lage des Kopfes des Patienten P in Übereinstimmung zu bringen und auch die Bilddarstellung auf der Anzeigeeinrichtung 18 entsprechend anzupassen. Wird das Element 15 des Patientenstuhls 12 beispielsweise im Zuge der Behandlung des Patienten P relativ zu dem Element 14 verstellt, worin ein Veränderung der Lage des Kopfes des Patienten P resultiert, wird dies über das Navigationssystem registriert und die Bilddarstellung kann optional an die veränderte Lage des Kopfes des Patienten P durch den Bildrechner 17 angepasst werden.

Des weiteren besteht mit Hilfe des Navigationssystems die Möglichkeit, durch die Bestimmung der Position des Instrumentes 27 relativ zu dem Patientenstuhl 12 und somit relativ zum Patienten P und dem 3D-Bilddatensatz in dem Referenzkoordinatensystem KR ein Abbild des Instrumentes 27 in ein mit der Röntgeneinrichtung gewonnenes 3D-Bild vom Kopf- bzw. Kieferbereich des Patienten P einzublenden und auf diese Weise den chirurgischen Eingriff zu unterstützen. Dies ist besonders dann vorteilhaft, wenn beispielsweise die Spitze des Instrument 27 durch das Eindringen in den Kieferbereich des Patienten P während der Operation nicht mehr sichtbar ist.

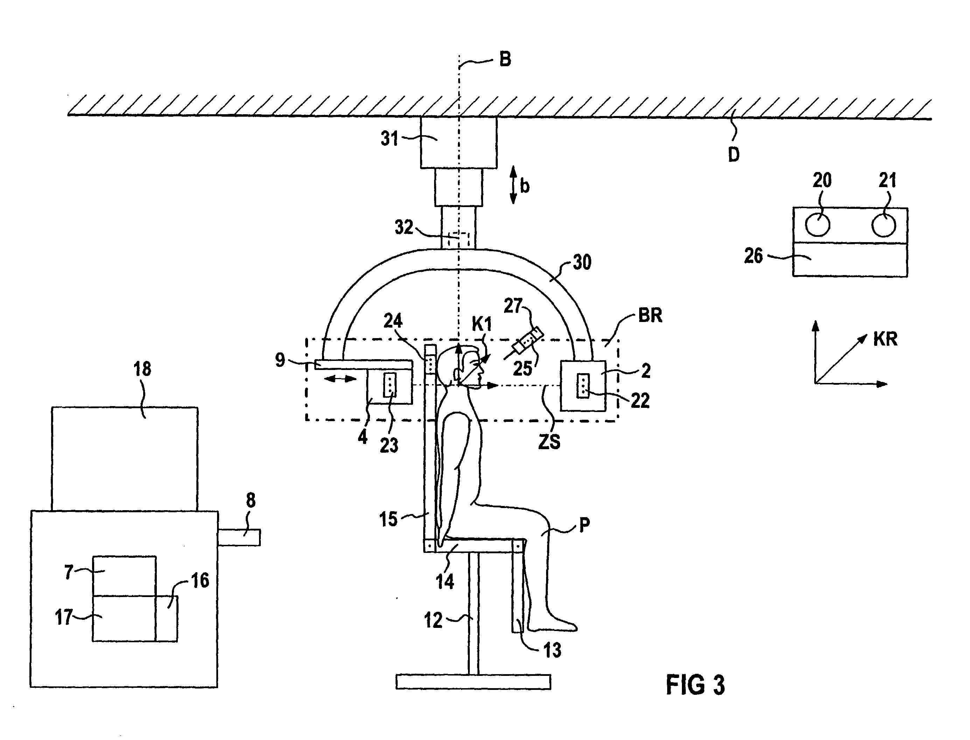

Die Fig. 3 zeigt einen zweiten, erfindungsgemäß ausgebildeten medizinischen Arbeitsplatz für die Diagnostik und für chirurgische Eingriffe im Mund-, Kiefer- oder Gesichtsbereich des Patienten P. Der in Fig. 3 dargestellte medizinische Arbeitsplatz ist bis auf Teile der Röntgeneinrichtung mit dem in Fig. 1 gezeigten und vorstehend beschriebenen Arbeitsplatz bau- und funktionsgleich. Im Unterschied zu dem in Fig. 1 gezeigten medizinischen Arbeitsplatz weist die Röntgeneinrichtung des in Fig. 3 gezeigten medizinischen Arbeitsplatzes einen um eine vertikal verlaufende Achse B schwenkbaren deckengehängten C-Bogen 30 auf, an dem einander gegenüberliegend eine Röntgenstrahlenquelle 2 und ein Röntgenstrahlendetektor 4 in definierter Weise angeordnet sind. Der Zentralstrahl ZS eines von der Röntgenstrahlenquelle 2 ausgehenden, kegelförmigen Röntgenstrahlenbündels trifft dabei annähernd mittig auf den Eingangsschirm des Röntgenstrahlendetektors 4. Der Röntgenstrahlendetektor 4 ist über die Verstelleinrichtung 9 derart an dem C-Bogen 30 angeordnet, dass er in Richtung des Zentralstrahlen ZS in geometrisch bestimmter Weise verstellbar ist. Der C-Bogen 30 ist über eine teleskopartig ausgebildete Halterung 31 an der Decke D des Behandlungszimmers angeordnet und mittels nicht dargestellter, durch den Steuerrechner 7 angesteuerter elektrischer Antriebe vertikal in die Richtungen des Doppelpfeiles b verstellbar. Mittels eines ebenfalls von dem Steuerrechner 7 angesteuerten Schrittmotors 32 kann der C-Bogen 30 relativ zu der Halterung 31 um die Achse B geschwenkt werden.

Zur Gewinnung eines 3D-Bilddatensatzes vom Kopf- bzw. Kieferbereich des Patienten P wird der C-Bogen 30 angetrieben durch den Schrittmotor 32, um die Achse B geschwenkt, wobei eine Serie von 2D-Projektionen bei unterschiedlichen Projektionswinkeln aufgenommen wird. Die in dem Bildspeicher 16 zwischengespeicherten 2D-Projektionen verwendet der Bildrechner 17 anschließend zusammen mit den vom Steuerrechner 7 zur Verfügung gestellten Projektionsgeometrien wie im Falle des in Fig. 1 beschriebenen Ausführungsbeispieles zur Erzeugung eines 3D-Bilddatensatzes vom Kopf- bzw. Kieferbereich des Patienten P. Die Projektionsgeometrien ermittelt der Steuerrechner 7 dabei aus den Steuerdaten des Schrittmotor 32, wobei dem Steuerrechner 7 die Ausgangslage des Röntgensystems aus den Steuerdaten des Schrittmotors 32, aus den Steuerdaten der elektrischen Antriebe der Halterung 31 und den Einstelldaten der Verstelleinrichtung 9 bekannt ist. Die Lage des 3D-Bilddatensatzes in dem Röntgenkoordinatensystem K1 ist aufgrund der bekannten Positionen des Röntgensystems bei der Aufnahme der 2D-Projektionen ebenfalls bekannt, so dass eine direkte Übertragung von anhand von 3D-Bildern gewonnenen Planungsergebnissen auf den Patienten P möglich ist.

Mit Hilfe des Navigationssystems kann wie in zuvor beschriebener Weise eine Beziehung zwischen dem Röntgenkoordinatensystem K1 und dem Referenzkoordinatensystem KR hergestellt werde, so dass bei Verstellungen des Patientenstuhles 12 die Lage des 3D-Bilddatensatzes mit der Lage des Kopfes des Patienten P in Übereinstimmung gebracht und Navigation des Instrumentes 27 betrieben werden kann.

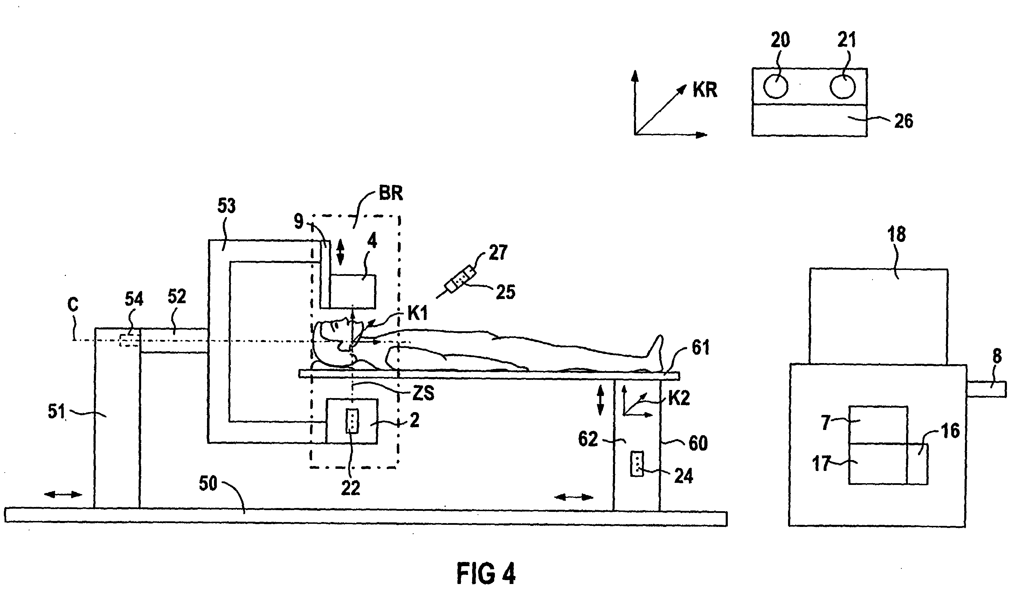

In Fig. 4 ist ein drittes Ausführungsbeispiel eines erfindungsgemäßen medizinischen Arbeitsplatzes mit einer Röntgeneinrichtung und einer Patientenlagerungseinrichtung gezeigt. Komponenten des Arbeitsplatzes, welche mit Komponenten des in Fig. 1 dargestellten Arbeitsplatzes wenigstens im wesentlichen bau- und funktionsgleich sind, sind mit gleichen Bezugszeichen versehen.

Die Röntgeneinrichtung umfasst eine in einer Schiene 50 motorisch definiert verstellbare Standsäule 51, an der eine Halterung 52 zur Aufnahme einer U-förmigen Tragevorrichtung 53 angeordnet ist. An der U-förmigen Tragevorrichtung 53 sind die Röntgenstrahlenquelle 2 und der Röntgenstrahlendetektor 4 in definierter Weise derart einander gegenüberliegend angeordnet, dass ein von der Röntgenstrahlenquelle 2 ausgehender Zentralstrahl ZS eines kegelförmigen Röntgenstrahlenbündels annähernd mittig auf den Eingangsschirm des Röntgenstrahlendetektors 4 trifft. Wie bei den zuvor beschriebenen Ausführungsbeispielen ist der Röntgenstrahlendetektor 4 an der Verstellvorrichtung 9 angeordnet, an der er in Richtung des Zentralstrahles ZS definiert verstellbar ist. Die Halterung 52 der U-förmigen Tragevorrichtung 53 ist zusammen mit der Tragevorrichtung 53 relativ zu der Standsäule 51 um eine wenigstens im wesentlichen horizontal durch die U-förmige Tragevorrichtung verlaufende Achse C, bei der es sich um die sogenannte Angulationsachse handelt, schwenkbar.

Im Falle des in Fig. 4 beschriebenen Ausführungsbeispiels ist der Patient P auf einer Patientenliege 60 gelagert, welche eine Patientenlagerungsplatte 61 und eine Teleskopsäule 62 aufweist. Die Teleskopsäule 62 ist ebenfalls in nicht dargestellter Weise in der Schiene 50 motorisch verstellbar gelagert. Die Steuerung der motorischen Verstellung der Standsäule 51 sowie der Teleskopsäule 62 in der Schiene 50, wie auch vertikal, erfolgt durch den Steuerrechner 7, welchem demzufolge die Positionen der Röntgeneinrichtung und der Patientenlagerungseinrichtung relativ zueinander bekannt sind.

Zur Gewinnung eines 3D-Bilddatensatzes vom Kopf- bzw. Kieferbereich des Patienten P wird die Tragevorrichtung 53 angetrieben durch einen von dem Steuerrechner 7 angesteuerten Schrittmotor 54, um die Achse C geschwenkt, wobei eine Serie von 2D-Projektionen bei unterschiedlichen Projektionswinkeln aufgenommen wird. Die in dem Bildspeicher 16 zwischengespeicherten 2D-Projektionen verwendet der Bildrechner 17 anschließend zusammen mit den vom Steuerrechner 7 zur Verfügung gestellten Projektionsgeometrien, welche aus den Steuerdaten des Schrittmotors 54 gewonnen werden, wie im Falle der in Fig. 1 und 3 gezeigten Ausführungsbeispiele zur Erzeugung eines 3D-Bilddatensatzes vom Kopf- bzw. Kieferbereich des Patienten P. Die Lage des 3D-Bilddatensatzes in dem Röntgenkoordinatensystem K1 ist dabei aufgrund der bekannten Positionen des Röntgensystems bei der Aufnahme der 2D-Projektionen wiederum bekannt. Eine Übertragung von anhand von 3D-Bildern gewonnenen Planungsergebnissen direkt auf den Patienten P ist demnach möglich.

Da es sich bei dem schienenbasierten System der Verstellung der Röntgeneinrichtung und der Patientenlagerungsvorrichtung um ein präzises mechanisches Verschiebesystem handelt, bei dem dem Steuerrechner 7 sowohl die Positionsdaten der Röntgeneinrichtung als auch der Patientenlagerungseinrichtung bekannt sind, kann der Bildrechner 17 anhand der Positionsdaten des Steuerrechners 7 eine Beziehung zwischen dem willkürlich wählbaren Koordinatensystem K1 der Röntgeneinrichtung und einem willkürlich wählbaren Koordinatensystem K2 der Patientenlagerungsvorrichtung auch ohne Navigationssystem herstellen. Auf diese Weise kann bei einer Veränderung der Lage des Patienten P durch Verstellbewegungen der Patientenliege 60 der 3D-Bilddatensatz von dem Bildrechner 17 in Übereinstimmung mit der Lage des Kopfes des Patienten P gebracht werden und eventuell die Bilddarstellung angepasst werden.

Zusätzlich kann an dem medizinischen Arbeitsplatz das in den in Fig. 1 und 3 gezeigte und vorstehend beschriebene Navigationssystem vorhanden sein, um Abbilder des Instrumentes 27 in mit der Röntgeneinrichtung erzeugte 3D-Bilder vom Mund- oder Kieferbereich des Patienten P einblenden zu können. Da dem Steuerrechner 7 aufgrund des mechanischen Verschiebesystems die Positionen der Röntgeneinrichtung und der Patientenlagerungseinrichtung relativ zueinander bekannt sind, genügt es nur die Röntgeneinrichtung oder die Patientenlagerungseinrichtung mit einem Referenzelement 22 bzw. 24 zu versehen, um die Positionen der Röntgeneinrichtung und der Patientenlagerungseinrichtung in dem Referenzkoordinatensystem KR zu bestimmen und Navigation des Instrumentes 27 betreiben zu können. Die hierfür erforderlichen Positionsdaten stellen der Rechner 26 und der Steuerrechner 7 dem Bildrechner 17 zur Verfügung, so dass dieser Abbilder des Instrumentes 27 in aus dem 3D-Bilddatensatz gewonnene 3D-Bilder realitätsgetreu einblenden kann.

Bei dem vorstehend beschriebenen Navigationssystem muss es sich im übrigen nicht notwendigerweise um ein optisches Navigationssystem handeln. Vielmehr können auch mechanische, akustische oder elektromagnetische Navigationssysteme verwendet werden.

Die elektrischen Verbindungen zwischen den Komponenten der jeweiligen Arbeitsplätze, beispielsweise die Verbindungen zwischen den Schrittmotoren und dem Steuerrechner 7 oder dem Rechner 26 und dem Bildrechner 17, sind in den Fig. 1 bis 4 nicht dargestellt, da sie in an sich bekannter Weise ausgeführt sind.

Die beschrieben Ausführungsbeispiele der erfindungsgemäßen Röntgeneinrichtung sowie des erfindungsgemäßen Arbeitsplatzes sind im übrigen nur exemplarisch zu verstehen. Details der Röntgeneinrichtung bzw. des Arbeitsplatzes können demnach im Rahmen der Erfindung auch anders ausgeführt sein.

Darüber hinaus sind Mischformen der in den Fig. 1 bis 4 gezeigten Ausführungsbeispiele möglich.

## Patentansprüche

1. Röntgeneinrichtung für radiologische Aufnahmen im Kopf- und Kieferbereich eines Patienten (P) mit einer Röntgenstrahlenquelle (2) und einem Röntgenstrahlendetektor (4), welche derart an einer U-förmig oder C-bogenförmig ausgebildeten Tragevorrichtung (30, 53) einander gegenüberliegend angeordnet sind, dass der Zentralstrahl (ZS) eines von der Röntgenstrahlenquelle (2) ausgehenden kegelförmigen Röntgenstrahlenbündels annähernd mittig auf den Röntgenstrahlendetektor (4) trifft,
**dadurch gekennzeichnet, dass**
- die Röntgeneinrichtung Mittel (7, 10, 11, 32, 54) zur motorischen Verstellung der Tragevorrichtung (30, 53) um eine Achse (B, C) zur Aufnahme einer Serie von 2D-Projektionen vom Kopf- oder Kieferbereich des Patienten (P) aufweist, wobei die Achse (B, C) durch die Tragevorrichtung (30, 53) verläuft und die Tragevorrichtung (30, 53) um die Achse (B, C) motorisch schwenkbar ist, und
- die Röntgeneinrichtung Mittel (7, 16, 17) zur Erzeugung eines 3D-Bilddatensatzes aus den aufgenommenen 2D-Projektionen umfasst.

2. Röntgeneinrichtung nach Anspruch 1, bei dem die Mittel zur motorische Verstellung der Tragevorrichtung (30, 53) wenigstens einen digital gesteuerten Antrieb umfassen.

3. Röntgeneinrichtung nach Anspruch 2, bei dem der Antrieb einen Schrittmotor (10, 11, 32, 54) umfasst.

4. Röntgeneinrichtung für radiologische Aufnahmen im Kopf- und Kieferbereich eines Patienten (P) mit einer Röntgenstrahlenquelle (2) und einem Röntgenstrahlendetektor (4), welche derart einander gegenüberliegend angeordnet werden können oder angeordnet sind, dass der Zentralstrahl (ZS) eines von der Röntgenstrahlenquelle (2) ausgehenden kegelförmigen Röntgenstrahlenbündels annähernd mittig auf den Röntgenstrahlendetektor (4) trifft und mit Mitteln (1, 3, 9, 30, 53) zur Halterung der Röntgenstrahlenquelle (2) und des Röntgenstrahlendetektors (4),
**dadurch gekennzeichnet, dass**
- die Röntgeneinrichtung Mittel (7, 10, 11, 32, 54) zur motorischen Verstellung der Mittel (1, 3, 9, 30, 53) zur Halterung um eine Achse (A) zur Aufnahme einer Serie von 2D-Projektionen vom Kopf- oder Kieferbereich des Patienten (P) aufweist,
- die Mittel zur Halterung der Röntgenstrahlenquelle (2) und des Röntgenstrahlendetektors (4) zwei Stative (1, 3) umfassen, wobei die Röntgenstrahlenquelle (2) und der Röntgenstrahlendetektor (4) jeweils an einem der Stative (1, 3) angeordnet sind, welche um die Achse (A) und relativ zueinander motorisch verstellbar sind und
- die Röntgeneinrichtung Mittel (7, 16, 17) zur Erzeugung eines 3D-Bilddatensatzes aus den aufgenommenen 2D-Projektionen aufweist.

5. Röntgeneinrichtung nach Anspruch 4, bei dem die Mittel zur motorische Verstellung der Mittel (1, 3, 9, 30, 53) zur Halterung wenigstens einen digital gesteuerten Antrieb umfassen.

6. Röntgeneinrichtung nach Anspruch 5, bei dem der Antrieb einen Schrittmotor (10, 11, 32, 54) umfasst.

7. Röntgeneinrichtung nach einem der Ansprüche 1 bis 6, bei dem der Röntgenstrahlendetektor (4) in Richtung des Zentralstrahls (ZS) verstellbar ist.

8. Medizinischer Arbeitsplatz für die Diagnostik und für chirurgische Eingriffe im Mund-, Kiefer- oder Gesichtsbereich eines Patienten (P) aufweisend eine Röntgeneinrichtung nach einem der Ansprüche 1 bis 7, eine Patientenlagerungseinrichtung (60) und Mittel (7) zur Bestimmung der Positionen der Röntgeneinrichtung und der Patientenlagerungseinrichtung (60) relativ zueinander, wobei die Röntgeneinrichtung und die Patientenlagerungseinrichtung (60) in definierter Weise relativ zueinander angeordnet und in definierter Weise relativ zueinander mechanisch verstellbar sind.

9. Medizinischer Arbeitsplatz nach Anspruch 8 aufweisend ein Navigationssystem (20 bis 26).

10. Medizinischer Arbeitsplatz für die Diagnostik und für chirurgische Eingriffe im Mund-, Kiefer- oder Gesichtsbereich eines Patienten (P) aufweisend eine Röntgeneinrichtung nach einem der Ansprüche 1 bis 7, eine Patientenlagerungseinrichtung (12) und ein Navigationssystem (20 bis 26) zur Bestimmung der Positionen der Röntgeneinrichtung und der Patientenlagerungsvorrichtung (12) relativ zueinander.

11. Medizinischer Arbeitsplatz nach Anspruch 9 oder 10 aufweisend wenigstens ein medizinisches Instrument (27), dessen Positionen mit dem Navigationssystem (20 bis 26) bestimmbar sind, und mit Mitteln (17) zur Einblendung eines Abbildes des Instrumentes (27) in ein mit der Röntgeneinrichtung gewonnenes Bild zur Unterstützung chirurgischer Eingriffe.

## Claims

1. x-ray device for recording radiological images in the head and jaw area of a patient (P) with an x-ray source (2) and an x-ray detector (4), which are arranged opposite each other on a U-shaped or C-arm shaped design of carrier device (30, 53) in such a way that the central ray (ZS) of a conical bundle of rays emitted by the x-ray source (2) strikes the x-ray detector (4) in approximately the center,
**characterized in that**
- The x-ray device features means (7, 10, 11. 32, 54) for motorized adjustment of the carrier device (30, 53) about an axis (H, C) to record a series of 2D projections of the head or jaw area of the patient (P), whereby the axis (B, C) runs through the carrier device (30, 53) and the carrier device (30;;53) can be rotated around the axis (B, C) by a motor-driven mechanism, and
- The x-ray device comprises means (7, 16, 17) for creation of a set of 3D image data from the recorded 2D projections.

2. x-ray device according to Claim 1, in which the means for motorized adjustment of the carrier device (30, 53) comprise at least one digitally-controlled drive.

3. x-ray device according to Claim 2, in which the drive comprises a stepping motor (10, 11, 32, 54).

4. X-ray device for recording radiological images in the head and jaw area of a patient (P) with an x-ray source (2) and an x-ray detector (4), which can be or are arranged opposite each other in such a way that the central ray (ZS) of a conically-shaped bundle of rays emitted by the x-ray source (2) strikes the x-ray detector (4) in approximately the center and with means (1, 3, 9, 30, 53) for mounting the x-ray source (2) and the x-ray detector (4),
**characterized in that**
- The x-ray device features means (7, 10, 11, 32, 54) for motorized adjustment of the means (1, 3, 9, 30, 53) for mounting about an axis (A) to record a series of 2D projections from the head or jaw area of the patient (P),
- The means for mounting the x-ray source (2) and the x-ray detector (4) comprise two stands (1, 3), with the x-ray source S and x-ray detector (4) each being arranged on one of the stands (1, 3) which can be adjusted by a motor about the axis (A) and relative to each other and
- the x-ray device features means (7, 16, 17) to create a set of 3D image data from the recorded 2D projections.

5. x-ray device according to Claim 4, in which the means for motorized adjustment of the means (1, 3, 9, 30, 53) for mounting comprises at least one digitally-controlled drive.

6. x-ray device according to Claim 5, in which the drive comprises a stepping motor (10, 11, 32, 54).

7. x-ray device according to one of Claims 1 to 6, in which the x-ray detector (4) can be adjusted in the direction of the central ray (ZS).

8. Medical workstation for diagnostics and for surgical procedures in the mouth, jaw or face area of a patient (P) featuring an x-ray device according to one of the Claims 1 to 7, a patient support device (60) and means (7) for determining the positions of the x-ray device and patient support device (60) relative to each other, whereby the x-ray device and the patient support device (60) can be arranged in a defined way relative to each other and can be mechanically adjusted in a defined way relative to each other.

9. Medical workstation according to Claim 9 featuring a navigation system (20 to 26).

10. Medical workstation for diagnostics and for surgical procedures in the mouth, jaw or face area of a patient (P) featuring an x-ray device according to one of the Claims 1 to 7, a patient support device (12) and a navigation system (20 to 26) for determining the positions of the x-ray device and patient support device (12) relative to each other.

11. Medical workstation according to Claim 9 or 10, featuring at least one medical Instrument (27) of which the positions can be determined with the navigation system (20 to 26) and with means (17) for incorporating an image of the instrument (27) into an image obtained with the x-ray device for supporting surgical procedures.

## Revendications

1. Appareil de radiographie pour des enregistrements radiologiques dans la région de la tête et de la mâchoire d'un patient (P), comportant une source (2) de rayons X et un détecteur (4) de rayons X qui sont disposés, sur un dispositif (30, 53) porteur réalisé en forme de U ou en forme d'arc en C, en vis-à-vis de telle sorte que le rayon (ZS) central d'un faisceau conique de rayons X partant de la source (2) de rayons X rencontre approximativement en son milieu le détecteur (4) de rayons X,
**caractérisé en ce que**
- l'appareil de radiographie comporte des moyens (7, 10, 11, 32, 54) pour le déplacement motorisé du dispositif (30, 53) porteur autour d'un axe (B, C) afin d'enregistrer une série de projections en 2 dimensions de la région de la tête ou de la mâchoire du patient (P),
l'axe (B, C) passant par le dispositif (30, 53) porteur et le dispositif (30, 53) porteur pouvant pivoter de façon motorisée autour de l'axe (B, C), et
- l'appareil de radiographie comprend des moyens (7, 16, 17) pour produire un jeu de données d'image en 3 dimensions à partir des projections en 2 dimensions enregistrées.

2. Appareil de radiographie suivant la revendication 1, dans lequel les moyens pour le déplacement motorisé du dispositif (30, 53) porteur comprennent au moins un entraînement à commande numérique.

3. Appareil de radiographie suivant la revendication 2, dans lequel l'entraînement comprend un moteur (10, 11, 32, 54) pas à pas.

4. Appareil de radiographie pour des enregistrements radiologiques dans la région de la tête et de la mâchoire d'un patient (P), comportant une source (2) de rayons X et un détecteur (4) de rayons X qui peuvent être disposés ou sont disposés en vis-à-vis de telle sorte que le rayon (ZS) central d'un faisceau conique de rayons X partant de la source (2) de rayons X rencontre approximativement en son milieu le détecteur (4) de rayons X, et comportant des moyens (1, 3, 9, 30, 53) de support de la source (2) de rayons X et le détecteur (4) de rayons X,
**caractérisé en ce que**
- l'appareil de radiographie comprend des moyens (7, 10, 11, 32, 54) pour le déplacement motorisé des moyens (1, 3, 9, 30, 53) de support autour d'un axe (A) afin d'enregistrer une série de projections en 2 dimensions de la région de la tête ou de la mâchoire du patient (P),
- les moyens de support de la source (2) de rayons X et du détecteur (4) de rayons X comprennent deux colonnes (1, 3), la source (2) de rayons X et le détecteur (4) de rayons X étant chacun disposés sur une des colonnes (1, 3), qui peuvent être déplacées de façon motorisée autour de l'axe (A) et l'une par rapport à l'autre, et
- l'appareil de radiographie comprend des moyens (7, 16, 17) pour produire un jeu de données d'image en 3 dimensions à partir des projections en 2 dimensions enregistrées.

5. Appareil de radiographie suivant la revendication 4, dans lequel les moyens pour le déplacement motorisé des moyens (1, 3, 9, 30, 53) de support comprennent au moins un entraînement à commande numérique.

6. Appareil de radiographie suivant la revendication 5, dans lequel l'entraînement comprend un moteur (10, 11, 32, 54) pas à pas.

7. Appareil de radiographie suivant l'une des revendications 1 à 6, dans lequel le détecteur (4) de rayons X peut être déplacé dans la direction du rayon (ZS) central.

8. Poste opératoire médical pour le diagnostic et pour des interventions chirurgicales dans la région de la bouche, de la mâchoire ou du visage d'un patient (P), comportant un appareil de radiographie suivant l'une des revendications 1 à 7, un dispositif (60) de soutien du patient et des moyens (7) pour déterminer les positions de l'appareil de radiographie et du dispositif (60) de soutien du patient l'un par rapport à l'autre, l'appareil de radiographie et le dispositif (60) de soutien du patient étant disposés l'un par rapport à l'autre d'une manière définie et pouvant être déplacés mécaniquement l'un par rapport à l'autre d'une manière définie.

9. Poste opératoire médical suivant la revendication 8, comportant un système (20 à 26) de navigation.

10. Poste opératoire médical pour le diagnostic et pour des interventions chirurgicales dans la région de la bouche, de la mâchoire ou du visage d'un patient (P), comportant un appareil de radiographie suivant l'une des revendications 1 à 7, un dispositif (12) de soutien du patient et un système (20 à 26) de navigation pour déterminer les positions de l'appareil de radiographie et du dispositif (12) de soutien du patient l'un par rapport à l'autre.

11. Poste opératoire médical suivant la revendication 9 ou 10, comportant au moins un instrument (27) médical dont les positions peuvent être déterminées par le système (20 à 26) de navigation, et comprenant des moyens (17) pour incruster une reproduction de l'instrument (27) dans une image obtenue avec l'appareil de radiographie, afin de faciliter des interventions chirurgicales.
